# EUROPÄISCHE PATENTANMELDUNG

(11) **EP 1 602 724 A1**
(43) Veröffentlichungstag der Anmeldung: **07.12.2005**
(21) Anmeldenummer: 05004213.4
(22) Anmeldetag: 06.06.1996
(51) Int. Cl.: C12N 15/11

(54) **Magnetisches Pigment**

(30) Priorität: 08.06.1995 DE 19520398; 12.10.1995 DE 19537985
(62) Teilanmeldung aus: 02016299.6
(71) Anmelder: Roche Diagnostics GmbH, 68305 Mannheim (DE)
(72) Erfinder: Kleiber, Jörg, 82377 Penzberg (DE); Walter, Thomas, 82377 Penzberg (DE); Harttig, Herbert, 67434 Neustadt (DE); Menning, Martin, 66287 Quierschied (DE); Lesniak, Christoph, 87474 Buchenberg (DE); Riedling, Michael, 82377 Penzberg (DE); Schmidt, Helmut K., 66130 Saarbrücken-Güdingen (DE)
(74) Vertreter: Weiss, Wolfgang

(57) **Zusammenfassung**

Gegenstand der Erfindung sind magnetische Partikel mit einer Glasoberfläche, Verfahren zur Reinigung eines biologischen Materials, insbesondere von Nukleinsäuren unter Verwendung von Glaspartikeln in Gegenwart chaotroper Salze, Verfahren zur Isolierung dieser biologischen Materialien und Verfahren zur Konzentrierung biologischen Materialien und die Überführung von biologischen Materialien aus Lösungen mit hoher Konzentration an Salzen in Lösungen mit niedriger Konzentration an Salzen.

SEQUENZPROTOKOLL

<110> Roche Diagnostics GmbH

<120> Magnetisches Pigment

<130> 18242 EP1

<140>
<141>

<150> DE19520398.4
<151> 1995-06-08

<150> DE19537985.3
<151> 1995-10-12

<150> EP96921935.1
<151> 1996-06-06

<160> 2

<170> PatentIn Ver. 2.1

<210> 1
<211> 34
<212> DNA
<213> Künstliche Sequenz

<220>
<223> Beschreibung der künstlichen Sequenz:
      Oligodeoxyribonucleotide

<400> 1
actgtgcttc ttgacccatg gcagaagcgc cttc                                34

<210> 2
<211> 34
<212> DNA
<213> Künstliche Sequenz

<220>
<223> Beschreibung der künstlichen Sequenz:
      Oligodeoxyribonucleotide

<400> 2
ccttcactgt ctgcctaact ccttcgtgtg ttcc                                34

## Beschreibung

Gegenstand der Erfindung sind magnetische Partikel mit einer Glasoberfläche, Verfahren zur Reinigung eines biologischen Materials, insbesondere von Nukleinsäuren unter Verwendung von Glaspartikeln in Gegenwart chaotroper Salze, Verfahren zur Isolierung dieser biologischen Materialien und Verfahren zur Konzentrierung biologischer Materialien und die Überführung von biologischen Materialien aus Lösungen mit hoher Konzentration an Salzen in Lösungen mit niedriger Konzentration an Salzen.

Manche biologische Materialien, insbesondere Nukleinsäuren, stellen im Hinblick auf ihre Isolierung aus der natürlichen Umgebung besondere Anforderungen. Zum einen sind sie oft in sehr geringen Konzentrationen vorhanden und zum anderen befinden sie sich oft in Nachbarschaft vieler anderer fester und gelöster Substanzen, die ihre Isolierung, beziehungsweise Bestimmung, beeinträchtigen.

In jüngerer Zeit hat es daher nicht an Versuchen gefehlt, Verfahren und Materialien zur Isolierung von Nukleinsäuren aus ihrer natürlichen Umgebung vorzuschlagen. Aus Proc. Natl. Acad. USA 76, 615 - 619 (1979) ist die Bindung von Nukleinsäuren aus Agarosegelen in Gegenwart von Natriumjodid in gemahlenem Flintglas beschrieben.

In Anal. Biochem. 121, 382 - 387 (1982) ist die Reinigung von Plasmid DNA aus Bakterien an Glasstaub in Gegenwart von Natriumperchlorat beschrieben.

In DE-A 37 34 442 ist die Isolierung von einzelsträngiger M13 Phagen-DNA an Glasfaserfiltern durch Ausfällung der Phagenpartikel mit Hilfe von Essigsäure und Lyse der Phagenpartikel mit Perchlorat beschrieben. Die an die Glasfaserfilter gebundenen Nukleinsäuren werden nach Waschen mit einem methanolhaltigen Puffer in Tris/EDTA-Puffer eluiert.

In Anal. Biochem. 175, 196- 201 (1988) ist ein ähnliches Verfahren zur Reinigung von DNA aus Lambdaphagen beschrieben.

Den bisher genannten Verfahren des Standes der Technik ist die selektive. Bindung von Nukleinsäuren an Glasoberflächen in chaotropen Salzlösungen gemeinsam, wobei die Nukleinsäure von Verunreinigungen wie Agarose, Proteinen oder Zelltrümmern abgetrennt wird. Zur Separierung der Glaspartikel von den Verunreinigungen wird nach dem Stand der Technik entweder Zentrifugation von Partikeln oder Durchsaugen von Flüssigkeiten durch Glasfaserfilter verwendet. Hierbei handelt es sich jedoch um einen limitierenden Schritt, der die Verarbeitung großer Probenzahlen stark behindert.

In Anal. Biochem. 201, 166 - 169 (1992) bzw. PCT GB 91/00212 ist die Verwendung von Magnetpartikeln zur Immobilisierung von Nukleinsäuren nach Ausfällung durch Zugabe von Salz und Ethanol beschrieben. Hierbei findet eine Agglutination der Nukleinsäuren unter Einschluss der Magnetpartikel statt. Das Agglutinat wird von dem ursprünglichen Lösungsmittel durch Anlegen eines Magnetfeldes und Waschen getrennt. Nach einem Waschschritt werden die Nukleinsäuren in einem Trispuffer gelöst. Dieses Verfahren hat jedoch den Nachteil, dass die Ausfällung nicht selektiv für Nukleinsäuren ist, sondern eine Vielzahl von festen und gelösten Stoffe mitagglutiniert werden. So ist es durch dieses Verfahren nicht möglich, eventuell vorhandene Inhibitoren für bestimmte enzymatische Reaktionen in ausreichendem Masse zu entfernen.

In US-A-4,233,169 ist ein poröses Glas beschrieben, das Magnetpartikel eingelagert enthält.

Auf dem Markt befindet sich derzeit auch sogenanntes magnetisches, poröses Glas, welches Magnetpartikelchen in einer porösen, partikulären Glasmatrix enthält, und wobei die Oberfläche von einer streptavidinhaltigen Schicht überzogen ist. Dieses Produkt kann zur Isolierung von biologischen Materialien, zum Beispiel Proteinen oder Nukleinsäuren verwendet werden, wenn diese in einem aufwendigen Vorbereitungsschritt so modifiziert werden, dass diese kovalent an Biotin gebunden sind.

Aufgabe der Erfindung war es, bessere Materialien zur Immobilisierung von biologischen Materialien und ein einfaches und für die Routine-Diagnostik geeignetes Verfahren zur Isolierung von biologischen Materialien, insbesondere Nukleinsäuren, bereitzustellen.

Gegenstand der Erfindung sind magnetische Partikel mit einer äußeren Glasoberfläche, die im wesentlichen porenfrei ist oder Poren eines Durchmessers von weniger als 10 nm aufweist. Ein weiterer Gegenstand sind ferromagnetische Partikel mit einer Glasoberfläche, Verfahren zur Isolierung biologischer Materialien, insbesondere von Nukleinsäuren, sowie Verfahren zur Herstellung magnetischer Glaspartikel.

Als Partikel bezeichnet der Fachmann feste Materialien mit einem geringen Durchmesser. Manchmal bezeichnet man solche Partikel auch als Pigmente. Im Sinne der vorliegenden Erfindung sind besonders Partikel geeignet, die eine durchschnittliche Korngröße von weniger als 100 µm haben. Besonders bevorzugt weisen sie eine durchschnittliche Korngröße von zwischen 10 und 60 um auf. Bevorzugt ist die Korngrößenverteilung relativ homogen, insbesondere liegen nahezu keine Teilchen < 10 µm oder > 60 µm vor.

Als magnetisch werden Materialien bezeichnet, die durch einen Magnet angezogen werden können, d. h. beispielsweise ferromagnetische oder superparamagnetische Materialien. Als magnetisch werden auch Materialien verstanden, die als weich magnetische Materialien bezeichnet werden, z. B. Ferrite. Besonders bevorzugt im Sinne der Erfindung sind ferromagnetische Materialien, insbesondere wenn sie noch nicht vormagnetisiert wurden. Unter Vormagnetisierung ist in diesem Zusammenhang das Inkontaktbringen mit einem Magneten zu verstehen, wodurch die Remanenz erhöht wird. Besonders bevorzugt sind ferromagnetische Materialien, wie z. B. Magnetit (Fe₃O₄) oder Fe₂O₃.

Unter einer äußeren Oberfläche eines Partikels wird die zusammenhängende Oberfläche verstanden, von der in Richtung auf die Umgebung des Partikels Senkrechte gebildet werden können, die dasselbe Partikel nicht noch einmal schneiden.

Unter eine Pore wird eine Ausnehmung in der äußeren Oberfläche des Partikels verstanden, bei denen die Oberfläche soweit in das Partikel hineinreicht, dass eine in der Ausnehmung auf der Oberfläche gebildete gedachte Senkrechte in Richtung auf die nächstliegende Umgebung des Partikels das Partikel mindestens 1mal schneidet. Poren reichen außerdem tiefer als ein Radius der Pore in das Partikel hinein.

Unter einem Glas im Sinne der vorliegenden Erfindung wird ein siliciumhaltiges amorphes Material verstanden. Das Glas kann weitere Materialien enthaltenen, z. B.

| | |
|---|---|
| B₂O₃ | (0 - 30 %), |
| Al₂O₃ | (0 - 20 %), |
| CaO | (0 - 20 %), |
| BaO | (0 - 10 %), |
| K₂O | (0 - 20 %), |
| N₂O | (0 - 20 %), |
| MgO | (0 - 18 %), |
| Pb₂O₃ | (0 - 15 %). |

In geringerem Umfang von 0 - 5 % können auch eine Vielzahl anderer Oxide, wie z. B. Mn₂O₃, TiO₂, As₂O₃, Fe₂O₃, CuO, CoO usw. enthalten sein. Als besonders wirksam haben sich Oberflächen einer Zusammensetzung von Borsilikatglas, Flintglas oder Silica erwiesen. Unter dem Gesichtspunkt der Ausbeute an Nukleinsäuren besonders bevorzugte Borsilikatgläser haben einen Boroxidgehalt von mehr als 25 %; als besonders wertvoll wurde ein Glas der Zusammensetzung SiO₂/B₂O₃ 70/30 erkannt. Besonders bevorzugt im Sinne der Erfindung sind Gläser, die durch den sogenannten Gel-Solprozess und anschließendes Trocknen und Verdichten der gebildeten Schicht gebildet werden. Dieser Prozess ist in seinen Grundzügen bekannt und wurde z. B. in C.J. Brinker, G.W. Scherer "Sol Gel science - The physics and chemistry of Sol Gel Processing", Academic Press Inc. 1990 und Sol-Gel Optics, Processing and Applications Lisa C. Klein Ed. Kluwer Academic Publishers 1994, Seite 450 ff. sowie in DE-A-1941191, DE-A-3719339, DE-A-4117041 und DE-A-4217432 beschrieben. Er wurde allerdings bisher noch nicht für magnetische Partikel beschrieben. Dass hiermit magnetische Partikel erzeugt werden können, die bei der Isolierung von biologischen Materialien, insbesondere Nukleinsäuren, ganz überraschende Eigenschaften haben, war nicht zu erwarten. Im Gel-Sol-Prozess werden Alkoxide von netzwerksbildenden Komponenten, z. B. SiO₂, B₂O₃, Al₂O₃, TiO₂, ZrO₂, GeO₂ zusammen mit Oxiden und Salzen anderer Komponenten, z. B. in alkoholischer Lösung, vorgelegt und hydrolysiert. In der Gleichung ist die Herstellung von einem Natriumboroaluminiumsilikatglas aufgeführt.

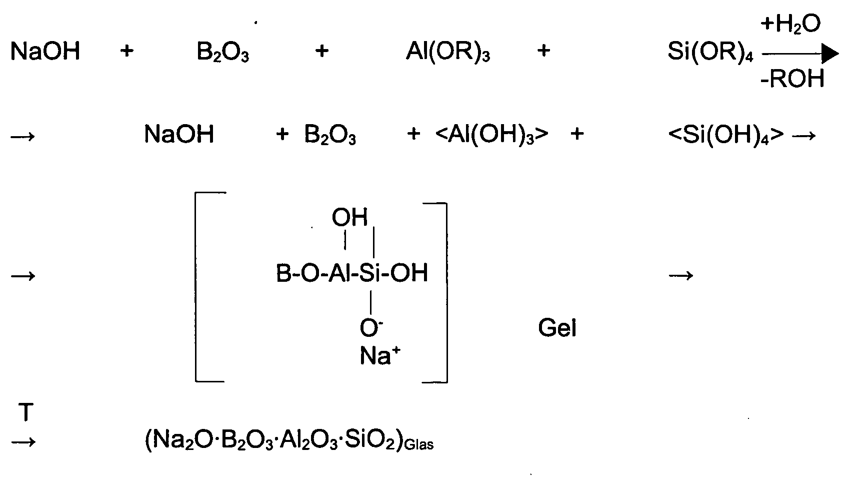

Durch die Zugabe von Wasser wird der Hydrolyseprozess der Ausgangskomponenten in Gang gesetzt. Die Reaktion verläuft relativ rasch, da die Alkaliionen katalytisch auf die Hydrolysegeschwindigkeit des Kieselsäureesters einwirken. Nach Ablauf der Gelbildung kann das entstehende Gel getrocknet und durch einen thermischen Prozess zu einem Glas verdichtet werden.

Das Mengenverhältnis Sol/Pigment hat einen erheblichen Einfluss auf die Ausbeute an erfindungsgemäßen magnetischen Pigment. Grenzen sind dadurch gegeben, dass der Pigmentanteil so gering ist, dass eine noch pump- und sprühfähige Masse entsteht. Bei zu geringem Pigmentanteil wird der Feinanteil, z. B. von nicht-magnetischem Material zu groß und stört. Als im Hinblick auf die Pigmentausbeute zweckmäßige Mengenverhältnisse wurden 10 bis 25 g Pigment/100 ml Sol gefunden.

Die Aufschlämmung wird zur Entstehung eines Pulvers bevorzugt durch eine Düse versprüht und das Aerosol auf einer Fallstrecke getrocknet. Die Düse wird bevorzugt geheizt, um die Trocknung der Aufschlämmung zu beschleunigen. Abhängig von der Geometrie der Düse beträgt die Düsentemperatur bevorzugt ca. 120 bis 200 °C. Ein Kompromiss wird gefunden durch ausreichende Verdampfungsgeschwindigkeit, jedoch Vermeiden von Verspritzen.

Im Hinblick auf die Ausbeute ist die Verdichtungstemperatur möglichst hoch zu wählen. Ist sie jedoch zu hoch, verkleben die Partikel untereinander und es bilden sich Agglomerate, die herausgesiebt werden sollten. Die Nachbehandlung unter Luft führt bei zu hohen Temperaturen zu einem Verlust der magnetischen Eigenschaften, weshalb zu hohe Temperaturen vermieden werden sollten.

Unter einer im wesentlichen porenfreien Oberfläche wird eine Oberfläche verstanden, die zu weniger als 5 %, bevorzugt weniger als 2 %, besonders bevorzugt weniger als 0,1 %, mit Poren der oben stehenden Definition durchsetzt ist. Sollten Poren vorhanden sein, so haben diese bevorzugt einen Durchmesser von weniger als 10, besonders bevorzugt 1 nm.

Besonders bevorzugt im Sinne der Erfindung sind Partikel, die einen Kern aus mit TiO₂ beschichteten Glimmer und darauf immobilisierten Magnetitpartikeln enthalten, wobei der so gebildete Verbundstoff von der Glasschicht umschlossen ist. Sowohl der Kern als auch die Magnetitpartikel sind kristallin und nicht porös. Die Räume auf der Oberfläche des Glimmers, welche nicht von den Magnetitpartikeln besetzt ist, sind von einer dickeren Glasschicht überzogen als die Spitzen der Magnetitpartikel, so dass sich eine im wesentlichen nicht-poröse Glasoberfläche ergibt.

Die Nichtporosität der magnetischen Partikel bezieht sich nur auf die äußere Oberfläche, nicht auf das Innere des Partikels, so dass das Partikel in seinem Inneren porös sein kann, wenn die Oberfläche nur von im wesentlichen porenfreiem Glas oder einer Glasoberfläche mit Poren eines Durchmessers von weniger als 10 nm umschlossen ist.

Überraschenderweise sind die erfindungsgemäßen magnetischen Partikel besonders vorteilhaft zur Isolierung biologischer Materialien aus Proben geeignet. Insbesondere werden lange Nukleinsäuren sehr wenig oder gar nicht zerstört, wenn sie daran immobilisiert werden. Das Material des Kerns ist darüber hinaus eine natürliche Ressource und somit ökologisch wenig bedenklich. Die Herstellung der erfindungsgemäßen Partikel ist darüber hinaus sehr wenig aufwendig und kostengünstig.

Ebenfalls Gegenstand der Erfindung sind ferromagnetische Partikel mit einer Glasoberfläche. Im Stand der Technik sind superparamagnetische Partikel beschrieben. Es hat sich nun herausgestellt, dass ferromagnetische Partikel, wenn sie mit einer Glasoberfläche überzogen sind, erhebliche Vorteile bei der Isolierung von biologischen Materialien aufweisen. Solange die ferromagnetischen Partikel noch keinem Magnetfeld ausgesetzt waren, sedimentieren sie ausschließlich unter Einfluss der Schwerkraft. Sie sind durch Schütteln einfach und schnell wieder zu suspendieren. Der Vorgang der Abscheidung ohne Magnetfeldeinfluss verläuft dabei bevorzugt langsamer als die Immobilisierung biologischer Materialien an ihrer Oberfläche. Dies gilt insbesondere für Nukleinsäuren. Die ferromagnetischen Partikel können auf einfache Weise mittels eines Magneten an einer bestimmten Stelle der Probenflüssigkeit gesammelt werden, um die Flüssigkeit von den Partikeln und somit den immobilisierten biologischen Materialien zu trennen.

Die Glasoberfläche der erfindungsgemäßen ferromagnetischen Partikel kann porenfrei sein oder aber Poren enthalten. Aus den oben genannten Gründen für die erfindungsgemäßen magnetischen Partikel ist es bevorzugt, dass die äußere Oberfläche auch der ferromagnetischen Partikel im wesentlichen porenfrei ist oder Poren eines Durchmessers von weniger als 10 nm aufweist. Auch die erfindungsgemäßen ferromagnetischen Partikel haben bevorzugt eine Korngröße zwischen 10 und 60 µm, besonders bevorzugt 20 und 50 µm. Besonders bevorzugt sind Partikel, bei denen eventuell in der Oberfläche vorhandene Poren einen Durchmesser von weniger als 10, besonders bevorzugt 1 nm haben. Ein Beispiel für einen erfindungsgemäßen ferromagnetischen Partikel ist der oben genannte Verbundstoff aus Glimmer- und Magnetitpartikeln, umschlossen von einer Glasschicht.

Ebenfalls Gegenstand der Erfindung ist ein Verfahren zur Isolierung eines biologischen Materials durch
- Inkontaktbringen einer Probe, die das biologische Material in einer Flüssigkeit enthält, mit den erfindungsgemäßen magnetischen Partikeln oder den erfindungsgemäßen ferromagnetischen Partikeln unter Bedingungen, bei denen das biologische Material an die Partikeloberfläche bindet, und
- Abtrennung des biologischen Materials von der Flüssigkeit.

Unter biologischen Materialien werden Materialien auf partikulärer oder molekularer Basis verstanden. Hierzu gehören insbesondere Zellen, z. B. Viren und Bakterien aber auch humane und tierische isolierte Zellen, wie Leukozyten, sowie immunologisch aktive nieder- und hochmolekulare chemische Verbindungen, wie Haptene, Antigene, Antikörper und Nukleinsäuren. Besonders bevorzugt sind Nukleinsäuren, z. B. DNA oder RNA.

Proben im Sinne der Erfindung sind beispielsweise klinische Proben, wie Blut, Serum, Mundspülflüssigkeit, Urin, Zerebralflüssigkeit, Sputum, Stuhl, Punktate und Knochenmarkproben. Die Probe kann auch aus dem Bereich der Umweltanalytik, der Lebensmittelanalytik oder der molekularbiologischen Forschung, z. B. aus Bakterienkulturen, Phagenlysaten und Produkten von Amplifikationsverfahren, z. B. PCR, stammen.

Erfindungsgemäß haben die magnetischen Partikel einen inneren Kern, auf den die äußere Glasoberfläche aufgebracht ist. Bei dem Kern kann es sich um einen Verbundstoff, jedoch auch um einfache Eisenkerne handeln. Der Kern kann auch aus einer kristallinen oder keramischen oder glasartigen Struktur bestehen, in die Eisenoxid eingelagert ist.

Mit dem geschilderten Verfahren kann natives oder modifiziertes biologisches Material isoliert werden. Unter nativem biologischem Material wird Material verstanden, dessen Struktur gegenüber den natürlich vorkommenden biologischen Materialien nicht irreversibel verändert wurde. Dies schließt jedoch nicht die Modifizierung anderer Bestandteile der Probe aus. Sollen beispielsweise Zellen isoliert werden, so kann zwar das die Zellen umgebende Medium modifiziert sein, nicht jedoch die Zellen als solche. Sollen Nukleinsäuren isoliert werden, so sollen auch diese in der nativen Form, d h. nicht denaturiert, geschnitten oder durch Ankoppelung reaktiver Gruppen modifiziert sein. Der Begriff natives biologisches Material umfasst daher insbesondere biotinylierte Nukleinsäuren nicht. Beispiele für native biologische Materialien sind Phagen-DNA oder zelluläre Nukleinsäuren aus Blut.

Modifizierte biologische Materialien umfassen Materialien, die nicht in der Natur vorkommen, z. B. Nukleinsäuren, die durch Anheftung reaktiver, nachweisbarer oder zur Immobilisierung befähigenden Gruppen modifiziert sind, z. B. biotinylierte Nukleinsäuren.

In bestimmten Fällen kann die Probe ohne Vorbehandlung in das erfindungsgemäße Isolierungsverfahren eingesetzt werden. In vielen Fällen sollte die Probe jedoch durch eine geeignete Methode aufgeschlossen und das in der Probe enthaltende biologische Material freigesetzt werden. Verfahren zum Aufschluss von Proben sind dem Fachmann bekannt und können chemischer, enzymatischer oder physikalischer Natur sein. Auch eine Kombination dieser Verfahren ist möglich. Beispielhaft genannt sei Lyse durch Ultraschall, Hochdruck oder durch Scherung, durch Alkali, Detergenzien oder chaotrope Salzlösungen, oder durch Einwirkung von Proteinasen oder Lipasen. Speziell im Hinblick auf die Aufschlussverfahren zum Erhalt von Nukleinsäuren wird auf Sambrook et al.: Molecular Cloning, A Laboratory Manual, 2nd Addition, Cold Spring Harbour Laboratory Press, Cold Spring Harbour, NY und Ausubel et al.: Current Protocols in Molecular Biology 1987, J. Viley and Sons, NY, verwiesen.

Die Probe kann neben dem zu isolierenden biologischen Material weitere Bestandteile, z. B. Zelltrümmer, Proteine, Salze und weitere nicht zu isolierende Stoffe in einer Flüssigkeit enthalten. Diese Probe, die bevorzugt das biologische Material in nativer Form enthält, wird unter Bedingungen, bei denen das gewünschte biologische Material an die Partikeloberfläche bindet, mit den Partikeln in Kontakt gebracht. Die Bedingungen hierfür hängen von der Art des biologischen Materials ab, sind jedoch prinzipiell bekannt. Sie richten sich auch nach der Art der Bindung, über die das biologische Material an die Oberfläche gebunden wird. Sollen beispielsweise immunologische Wechselwirkungen für die Bindung ausgenutzt werden, so müssen Bedingungen gewählt werden, die für die Bildung von Immunkomplexen geeignet sind. Für Nukleinsäuren ist im Fall der modifizierten Nukleinsäuren eine Bindung über die Gruppen der Nukleinsäuren möglich, die die Modifizierung darstellen, z. B. Biotin über die Bindung an mit Streptavidin beschichtete Oberflächen. Insbesondere bei Nukleinsäuren ist jedoch der Fall der direkten Bindung von Nukleinsäuren an Glas bevorzugt, unter anderem deshalb, weil sich eine Modifizierung der Nukleinsäuren erübrigt und schon native Nukleinsäuren gebunden werden können. Die Bindung nativer Nukleinsäuren an Glaspartikel kann analog zu Verfahren des Standes der Technik erfolgen. Bevorzugt erfolgt sie in Gegenwart chaotroper Salze, wobei die Konzentration dieser Salze zwischen 2 und 8 mol/l beträgt, bevorzugt 4 bis 6 mol/l. Bei chaotropen Salzen handelt es sich z. B. um Natriumjodit, Natriumperchlorat, Guanidininiumthiocyanat, Guanidiniumisothiocyanat oder Guanidiniumhydrochlorit, ist jedoch nicht auf diese Verbindungen beschränkt.

Zum Inkontaktbringen der Probe mit den Partikeln wird die Probe mit den Partikeln vermischt und für eine für die Bindung ausreichende Zeit inkubiert. Die Länge der Inkubation ist dem Fachmann in der Regel aus der Behandlung mit nicht-magnetischen Partikeln bekannt, eine Optimierung ist durch Durchführung einer Bestimmung der Menge an immobilisiertem biologischem Material an der Oberfläche zu verschiedenen Zeitpunkten möglich. Für Nukleinsäuren können Inkubationszeiten zwischen 10 Sekunden und 30 Minuten zweckmäßig sein.

Je nach Größe und Art der magnetischen Partikel findet schon während der Inkubationszeit eine Separation der Partikel von der Flüssigkeit statt oder erhält sich die Suspension über längere Zeit. Wenn die Partikel eine sehr kleine Korngröße aufweisen und superparamagnetisch sind, erhält sich die Suspension über einen längeren Zeitraum. Handelt es sich um Partikel mit einer größeren Korngröße, so findet bereits während der Inkubation eine langsame Separation der Partikel von der Flüssigkeit statt. Insbesondere wenn es sich um ferromagnetische Partikel handelt, bilden sich solche Aggregate. Für den bevorzugten Fall, dass die ferromagnetischen Partikel nicht vormagnetisiert sind, ist eine besonders schonende Separation gewährleistet.

Die Immobilisierung findet bevorzugt nicht durch Ausfällung durch Erniedrigung der Löslichkeit der zu immobilisierten Materialien statt. Stattdessen beruht die Immobilisierung auf biospezifischen Wechselwirkungen (Fang-Moleküle) oder Adsorption. Dies vermeidet weitgehend unspezifische Einschlüsse von Verunreinigungen.

Nach der Inkubation erfolgt die Abtrennung des biologischen Materials von der Flüssigkeit. Dies wird allgemein durch die Separation des an die magnetischen Partikel gebundenen Materials mit Hilfe eines Magnetfeldes erreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden wurden, entfernt werden. Diese Entfernung hängt von der Art des Gefäßes ab, in dem die Inkubation stattgefunden hat. Geeignete Verfahrensschritte sind Abpipettieren oder Absaugen der Flüssigkeit.

Danach können die magnetischen Partikel gewünschtenfalls ein- oder mehrmals mit einer Waschlösung gereinigt werden. Die Waschlösung wird so gewählt, dass eine Ablösung des biologischen Materials von der Partikeloberfläche möglichst nicht stattfindet, jedoch nicht zu isolierende Verunreinigungen möglichst gut weggewaschen werden. Dieser Waschschritt findet bevorzugt durch Inkubation der Waschlösung mit den Partikeln statt, wobei bevorzugt eine Resuspension der Partikel vorgenommen wird, z. B. durch Schütteln oder Anlegung eines nicht mit dem ersten Magnetfeld identischen Magnetfeldes. Die verunreinigte Waschlösung wird bevorzugt genauso entfernt wie die Probe in dem oben genannten Schritt zur Bindung des biologischen Materials.

Im Anschluss an den letzten Waschschritt kann ein kurzer Trocknungsschritt der magnetischen Partikel im Vakuum oder durch Ausdampfen (lassen) der Flüssigkeit vorgenommen werden, wobei auch eine Vorbehandlung mit Aceton möglich ist.

Das so gereinigte biologische Material kann, falls gewünscht, von den magnetischen Partikeln entfernt werden. Auch dieser Schritt richtet sich nach der Art der Bindung des biologischen Materials an die magnetischen Partikel. Für den Fall, dass es sich bei dem biologischen Material um native Nukleinsäuren und bei den magnetischen Partikeln um glasüberzogene Partikel handelt, kann die Nukleinsäure mittels eines Elutionspuffers mit niedrigem Salzgehalt von den erfindungsgemäßen Partikeln entfernt werden. Solche Puffer sind aus DE 3724442 und Analytical Biochemistry 175, 196-201 (1988) bekannt. Als Elutionspuffer mit niedrigem Salzgehalt werden insbesondere Puffer mit einem Gehalt von weniger als 0,2 mol/I eingesetzt. In einer besonders bevorzugten Ausführungsform enthält der Elutionspuffer Tris. In einer anderen besonderen Ausführungsform handelt es sich bei dem Elutionspuffer um entmineralisiertes Wasser.

In einer weiteren Ausführungsform kann das beschriebene Reinigungs- und Isolierungsverfahren im Anschluss an eine immunomagnetische Separation von Zellen (z. B. virale Partikel oder prokariontische bzw. eukariontische Zellen) aus einer Körperflüssigkeit oder einem Gewebe erfolgen. Hierzu wird die Probe mit magnetischen Partikeln, an welche ein Antikörper gegen ein Antigen auf der Zelle immobilisiert ist, z. B. unter Schütteln inkubiert. Solche Partikel können erfindungsgemäße Partikel sein, aber auch käufliche (z. B. MACS Microbeads der Firma Miltenyi Biotec GmbH, Bergisch Gladbach, BRD). Nach Anlegen eines Magnetfeldes erfolgen ein oder mehrere Waschschritte mit einer salzhaltigen Waschlösung. Man erhält Partikel, an welche die gewünschten Zellen gebunden sind. Schließlich werden die gebundenen Zellen in einem salzhaltigen Puffer resuspendiert. In einer bevorzugten Ausführungsform ist dieser salzhaltige Puffer eine chaotrope Salzlösung, so dass die in der Zelle vorhandenen Nukleinsäuren aus den Zellen freigesetzt werden.

Durch Kombination der oben beschriebenen Isolierung von Zellen mit der ebenfalls beschriebenen Isolierung von Nukleinsäuren, bevorzugt in ihrer nativen Form, an den erfindungsgemäßen magnetischen Partikeln, ergibt sich ein besonders vorteilhaftes Verfahren zur Isolierung von Nukleinsäuren aus zellhaltigen Proben. Vorteil dieser Ausführungsform ist die mögliche Einfachheit (Single-Tube-Methode), hohe Sensitivität (besonders wichtig in der medizinischen Mikrobiologie und Onkologie) und die leichte Automatisierbarkeit.

Die als Folge der erfindungsgemäßen Verfahren isolierten biologischen Materialien können nun in beliebiger Weise weiter verwendet werden. Beispielsweise können sie als Substrat für verschiedene enzymatische Reaktionen verwendet werden. Im Falle der Nukleinsäuren seien als Beispiel die Sequenzierung, die radioaktive oder nicht-radioaktive Markierung, die Amplifikation einer oder mehrerer in ihr enthaltender Sequenzen, die Transkription, die Hybridisierung mit markierten Sondennukleinsäuren, die Translation oder die Ligation genannt. Ein Vorteil des erfindungsgemäßen Verfahrens ist, dass die Abtrennung des biologischen Materials von der Flüssigkeit sehr einfach ist. Im Stand der Technik wurde zur Separierung von Glaspartikeln von Verunreinigungen entweder ein Zentrifugationsschritt angewandt, oder im Falle der Bindung des biologischen Materials an Glasfiberfilter die Flüssigkeit durch diese Flüssigkeit gesaugt. Hierbei handelt es sich um einen limitierenden Schritt, der die Verarbeitung von großen Probenzahlen behindert.

Mit den erfindungsgemäßen Partikeln ist eine effektivere Abtrennung der biologischen Materialien von Verunreinigungen möglich. Insbesondere können Inhibitoren für bestimmte enzymatische Reaktionen erfindungsgemäß in besonders gutem Umfang entfernt werden. Die Ausbeute an biologischem Material ist vergleichsweise hoch. Eine Fraktionierung langer Nukleinsäuren wurde nicht beobachtet. Die erfindungsgemäßen Partikel sind bevorzugt schneller magnetisierbar.

In Figur 1 ist schematisch eine Isolierung von Nukleinsäuren aus einer zellhaltigen Probe gezeigt.

In Figur 2 ist die Auftrennung erfindungsgemäß isolierter Nukleinsäuren in einem Agarosegel gezeigt.

In Figur 3 ist die Auftrennung von Reaktionsprodukten nach erfindungsgemäßer Isolierung und PCR-Amplifikation dargestellt.

In Figur 4 ist ein Gel der Ergebnisse aus Beispiel 4 gezeigt.

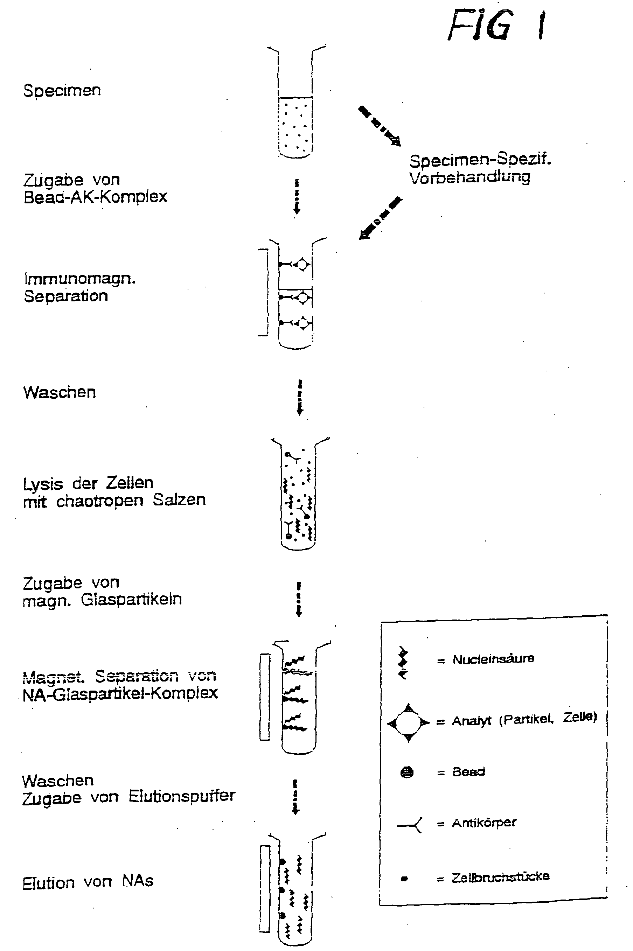

In Figur 1 ist eine Isolierung von Nukleinsäuren aus einer zellhaltigen Probe schematisch dargestellt. Die Probe (Specimen), welche Zellen enthält, wird probenspezifisch so vorbehandelt, dass die Zellen, in denen die Nukleinsäuren nachgewiesen werden sollen, in geeigneter Form vorliegen. Hierzu gehört z. B. bei Proben, welchen Körperflüssigkeiten entnommen wurden, die Zugabe von Reagenzien, z. B. zur Verflüssigung von zähflüssigen Proben, z. B. Speichelproben. Der so vorbereiteten Probe wird in einem Gefäß ein an eine Festphase, bevorzugt an eine Perle (Bead) gebundener Antikörper zugegeben, welcher die Zelle erkennen und binden kann. Als geeignete Partner für den Antikörper haben sich beispielsweise Antigene auf der Zelloberfläche erwiesen. Die Spezifität des Antikörpers kann sich nach der Spezifität der zu lösenden Analyseaufgabe richten. Wenn es sich bei der Festphase um die Wand des Gefäßes handelt, werden die Zellen direkt an die Wand gebunden. Für den Fall, dass es sich bei den Festphasen um eine Perle handelt, werden diese durch geeignete Separationsmethoden von der Flüssigkeit separiert. Dies kann beispielsweise durch Filtration geschehen. Im Falle von magnetischen Perlen ist eine Abtrennung durch Anlegen eines Magnetfeldes an die Außenwand des Gefäßes möglich. Die separierten Zellen werden mit einer Flüssigkeit gewaschen, um Verunreinigungen, welche den Nachweis stören würden, mit dem die Zellen umgebenden Medium zu entfernen. Bevorzugt werden Bedingungen eingesetzt, bei denen die Zellen weder von der Festphase gelöst noch zerstört werden. Anschließend findet die Zerstörung der Zellen statt, die sogenannte Lyse. Eine Möglichkeit ist durch die Behandlung der Zellen mit chaotropen Salzen gegeben. Andere Möglichkeiten sind die Einwirkung von Proteinasen und Detergenzien.

Zu der Lysemischung werden in der bevorzugten Ausführungsform die erfindungsgemäßen Partikel zugegeben. Nach einer geeigneten Einwirkungszeit, die über die Beladung der Oberfläche mit Nukleinsäuren optimiert werden kann, werden die Partikel von der sie umgebenden Flüssigkeit, die weitere und nicht nachzuweisende Zellbestandteile enthält, getrennt. Dies geschieht wiederum bevorzugt durch Anlegen eines magnetischen Feldes mittels eines Magneten an der Gefäßwand.

Um eventuell noch anhaftende Verunreinigungen zu entfernen, wird bevorzugt mit einer Flüssigkeit gewaschen, die so ausgewählt wird, dass die zu bestimmenden Nukleinsäuren sich nicht von der Glasoberfläche ablösen. Zur Entfernung der Nukleinsäuren von der Glasoberfläche wird ein sogenannter Elutionspuffer zugegeben, der Reagenzbedingungen aufweist, unter denen sich die Nukleinsäuren von der Glasoberfläche lösen. Dies sind insbesondere Niedrigsalzbedingungen. Je nach beabsichtigter Weiterbehandlung der Nukleinsäuren kann die Flüssigkeit nun von den Partikeln getrennt und weiterbearbeitet werden. Es ist bevorzugt, diese Abtrennung bei anliegendem Magnetfeld vorzunehmen, so dass die Partikel separiert vorliegen.

Die folgenden Beispiele erläutern die Erfindung näher.

### Beispiel 1

### Herstellung der erfindungsgemäßen magnetischen Partikel

Es wurden 6 verschiedene Sole verwendet. Die Herstellung der Sole wurde nach folgenden Schemata durchgeführt:
Sol 1 (SiO₂:B₂O₃ = 7:3):
Die Synthese wurde in einem 250 ml Rundkolben unter ständigem Rühren durchgeführt. 86,6 ml Tetraethylorthosilicat
+ 7 ml wasserfreies unvergälltes Ethanol
+ 14,1 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 37,8 ml Trimethylborat.
Anschließend wird das Sol 2 Stunden bei 50 °C gehalten. Danach erfolgt die Zugabe von
+ 14,1 ml 0, 15 M HCl

Sol 2(SiO₂:B₂O₃= 4:1):
Die Synthese wurde in einem 250 ml Rundkolben unter ständigem Rühren durchgeführt. 100,5 ml Tetraethylorthosilicat
+ 7 ml wasserfreies unvergälltes Ethanol
+16,3 ml 0,15 M HCl

Es entsteht ein zweiphasiges Gemisch, welches bei Raumtemperatur solange gerührt wird, bis es einphasig wird. Danach erfolgt tropfenweise die Zugabe von + 25,6 ml Trimethylborat.
Anschließend wird das Sol 2 Stunden bei 50 °C gehalten. Danach erfolgt die Zugabe von + 16,3 ml 0,15 M HCl
Sol 3 (SiO₂:B₂O₃ = 85:15):
Die Synthese wurde in einem 250 ml Rundkolben unter ständigem Rühren durchgeführt. 107,8 ml Tetraethylorthosilicat
+ 7 ml wasserfreies unvergälltes Ethanol
+ 17,5 ml 0,15 M HCl

Es entsteht ein zweiphasiges Gemisch, welches bei Raumtemperatur solange gerührt wird, bis es einphasig wird. Danach erfolgt tropfenweise die Zugabe von + 19,4 ml Trimethylborat.
Anschließend wird das Sol 2 Stunden bei 50 °C gehalten. Danach erfolgt die Zugabe von + 17,5 ml 0,15 M HCl
Sol 4 (SiO₂:B₂O₃ = 4:1; 2 Mol% P₂O₅):
Die Synthese wurde in einem 250 ml Rundkolben unter ständigem Rühren durchgeführt. 100,5 ml Tetraethylorthosilicat
+ 7 ml wasserfreies unvergälltes Ethanol
+ 16,3 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 25, 6 ml Trimethylborat
Anschließend wird das Sol 2 Stunden bei 50 °C gehalten. Danach erfolgt die Zugabe von + 16,3 ml 0,15 M HCl
+ 1,63 g P₂O₅

Sol 5 (SiO₂:B₂O₃ = 4:1 Mol% Al₂O₃):
Die Synthese wurde in einem 250 ml Rundkolben unter ständigem Rührem durchgeführt. 100,5 ml Tetraethylorthosilicat
+ 7 ml wasserfreies unvergälltes Ethanol
+ 16,3 ml 0, 15 M HCl

Es entsteht ein zweiphasiges Gemisch, welches bei Raumtemperatur solange gerührt wird, bis es einphasig wird. Danach erfolgt tropfenweise die Zugabe von
+ 25,6 ml Trimethylborat.
Anschließend wird das Sol 2 Stunden bei 50 °C gehalten. Danach erfolgt die Zugabe von + 16,3 ml 0,15 M HCl
+ 3,06 g AlCl₃

Sol 6 (SiO₂:B₂O₃ = 4:1 Mol % ZrO₂):
Die Synthese wurde in einem 250 ml Rundkolben unter ständigem Rühren durchgeführt. 100,5 ml Tetraethylorthosilicat
+ 7 ml wasserfreies unvergälltes Ethanol
+ 16,3 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 25,6 ml Trimethylborat
+ 5,15 ml Zirkon(IV)-propylat, 70 Gew.% Lsg in 1-Propanol
Anschließend wird das Sol 2 Stunden bei 50 °C gehalten. Danach erfolgt die Zugabe von + 16,3 ml 0,15 M HCl

Nach weiteren 2 Stunden bei 50 °C wurde in jeweils 150 ml der Sole 22,5 g Iriodin 600 (Black Mica) eingerührt und anschließend mit einem Sprühtrockner (Büchi 190, Mini Spray Dryer) beschichtet. Die Düsentemperatur des Sprühtrockners betrug 134 °C.

Das durch den Sprühtrockenprozess erhaltene Pulver wurde anschließend einer Temperaturbehandlung unter Stickstoffatmosphäre (90 I/h) unterzogen. Die Aufheizgeschwindigkeit betrug hierbei 1 k/min und die Haltezeit betrug 2 Stunden bei der Verdichtungstemperatur. Diese Temperatur lag bei der Beschichtung mit Sol 1 bei 750 °C, bei der Beschichtung mit Sol 2 bei 860 °C und bei den übrigen Beschichtungen bei 800 °C. Nach dem Sinterprozess wurde der Ofen abgeschaltet und das Pulver auf Raumtemperatur abgekühlt. Eventuell entstandene Agglomerate wurden mit einem 50 µm Sieb ausgesiebt.

### Beispiel 2

### Herstellung von GMP1, GMP2, GMP3 und GMP4

GMP1, GMP2, GMP3 und GMP4 sind Pigmente aus unterschiedlichen Herstellungs-Chargen, die aus Sol 1 aus Beispiel 1 in einem Prozess nach Beispiel 1 unter folgenden Bedingungen erhalten wurden:

| **Parameter** | **GMP1** | **GMP2** | **GMP3** | **GMP4** |
|---|---|---|---|---|
| Alterung des Sols (h) (30 °C) | 36 | 36 | 36 | 36 |
| Pigmentanteil des Sols (g/100 ml) | 5 | 15 | 8 | 20 |
| Luftstrom der Düse (%) | 100 | 100 | 100 | 100 |
| Luftdruck (bar) | 6 | 6 | 6 | 3 |
| Düsentemperatur (°C) | 135 | 120 | 130 | 143 |
| Verdichtungstemperatur (°C) | 534 | 534 | 534 | 615 |
| 02-Nachbehandlung (1 Stunde) | (300 °C) | (300 °C) | 300 °C) | (400 °C) |
| Ausbeute an Pigment | niedrig | hoch | mittel | hoch |
| DNA-Ausbeute | niedrig | hoch | hoch | hoch |

### Beispiel 3

### PCR Probenvorbereitunq aus humanen Vollblut mit magnetischen Glaspartikeln

### Isolierung der Nukleinsäure

Von drei Glasmagnetpartikel-Chargen (GMP2 - 4) wurden je ca. 10 mg in Eppendorf Reaktionsgefäßen vorgelegt. Die genauen Einwaagen sind in Tabelle 1 angegeben, es wurden Dreifach-Bestimmungen durchgeführt.

Zu je 200 µl aufgetautem Vollblut wurden 40 µl Proteinase K (20 mg/ml, hergestellt aus Lyophilisat) pipetiert und sofort gemischt. Anschließend wurden 200 µl Bindepuffer (6 M Guanidin-HCl, 10 mM Tris-HCl, 10 mM Harnstoff, 30 % Triton X-100, pH 4,4) zugegeben, gemischt und für 10 Minuten bei 70 °C inkubiert. Nach Zugabe von 200 µl i-Propanol wurde für 10 Sekunden auf dem Vortex-Mischer gemischt, die Probe für 20 Minuten bei Raumtemperatur inkubiert und abermals für 10 Sekunden wie vor gemischt. Die Magnetseparation erfolgte für wenigstens 30 Sekunden im Magnetpartikelseparator von Boehringer Mannheim (Id.-Nr. 1 641 794). Der Überstand wurde abgenommen und wie weiter unten beschrieben analysiert.

Die Magnetpartikel wurden mit jeweils 500 µl Wasch-Puffer (20 mM NaCl, 10 mM Tris-HCl, pH 7,5 (25 °C), 80 % Ethanol) durch 10 Sekunden mischen, 1 Minute Inkubation bei Raumtemperatur und 10 Sekunden mischen gewaschen und mit dem Magnetpartikelseparator an die Gefäßwand gezogen. Der Überstand wurde abgenommen und verworfen. Die Waschprozedur wurde wiederholt bis der Waschüberstand farblos war (insgesamt 4 x Waschen). Nun wurden die Nukleinsäuren 3 x mit jeweils 200 µl auf 70 °C vorgewärmten Elutionspuffer (10 mM Tris-HCI, pH 8,5) durch 10 Sekunden mischen, 10 Minuten Inkubation bei Raumtemperatur und 10 Minuten mischen eluiert.

### Aufarbeitung des Überstandes

Der Überstand nach der 1. Bindung an die magnetischen Glaspartikel wurde folgendermaßen auf Gehalt an Nukleinsäuren überprüft: Der Überstand wurde in ein Filter-Tube (Boehringer Mannheim, Id.-Nr. 1744003, z. B. enthalten in High Pure PCR Product Purification Kit) gegeben und für 1 Minute bei 8000 rpm in einer Eppendorf Tischzentrifuge zentrifugiert. Der Durchlauf wird verworfen und das Filter-Tube 2 x mit 500 pl Wasch-Puffer gewaschen (Zentrifugation wie vor). Das Filter-Tube wird kurz trocken zentrifugiert und dann mit 2 x 200 µl auf 70 °C vorgewärmten 1 x Elutionspuffer durch erneute Zentrifugation eluiert.

### Analyse der Eluate und des Probenüberstandes

50 µl der Eluate bzw. der über Filter-Tube aufgearbeiteten Überstände wurden mit 10 µl Proben-Puffer versetzt und davon 45 µl in einem 0,8 %igen Agarosegel elektrophoretisch bei 120 V für 90 Minuten aufgetrennt.

Verschiedene Verdünnungen der Eluate bzw. der aufgearbeiteten Überstände wurden bei 260 und 280 nm in einem Uvikon 710 (Kontron) spektroskopisch vermessen.

Zwei 5 µl Aliquots der Eluate wurden als Doppelbestimmung durch Expand TM Long Template PCR (Boehringer Mannheim, Id.-Nr. 1681834) mit spezifischen Primern für das menschliche tPA-Gen (erwartete Produktlänge 15 kb) überprüft.

| Mix I | pro Ansatz | Mix II | pro Ansatz |
|---|---|---|---|
| dNTP, je 100 mM | 1 µl | Expand™ Puffer, 10 x | 5 µl |
| Primer 1, 200 ng/µl | 1 µl | Expand™ Polymerase | 0,75 µl |
| Primer 2, 225 ng/µl | 1 µl | H₂O_{bidest}. | 19,25 µl |
| H₂O_{bidest.} | 17 µl | | |
| | 20 µl | | 25 µl |

Mix I wird in ein dünnwandiges PCR-Tube vorgelegt mit 5 µl Eluat versetzt und Mix II zugegeben. Der Ansatz wird kurz gemischt und mit 30 µl Mineralöl überschichtet. Die Ansätze werden in einem Perkin-Elmer Thermocycler 9600 mit folgenden Programm amplifiziert:

| | | |
|---|---|---|
| 2 Minuten | 92 °C | |
| 10 Sekunden | 92 °C | |
| 30 Sekunden | 65 °C | 10 Zyklen |
| 12 Minuten | 68 °C | |
| 10 Sekunden | 92 °C | |
| 30 Sekunden | 65 °C | 20 Zyklen |
| 12 Minuten + 20 Sekunden pro Zyklus | 68 °C | |
| 7 Minuten | 68 °C | |
| anschließend | 7 °C | |

Die 50 µl PCR Ansätze wurden mit 10 µl Proben-Puffer versetzt und davon 45 µl in einem 0,8 %igen Agarosegel elektrophoretisch bei 120 V für 90 Minuten aufgetrennt.

### Ergebnisse

**Tabelle 1:**

| Ausbeute an Nukleinsäuren mit magnetischen Glaspartikeln aus 200 µl Blut | | | | | | | | | | | |
|---|---|---|---|---|---|---|---|---|---|---|---|
| | | | Überstand 1:8 | | | | 1. Eluat 1:8 | | | | |
| | | | 260 nm | 280 nm | Ausbeute | 260/28 0 | 260 nm | 280 nm | Ausbeute | 260/28 0 | |
| GMP/2 | 12 mg | 1 | 0,021 | 0,013 | 1,7 µg | 1,6 | 0,171 | 0,164 | 13,7 µg | 1,0 | |
| | 10 mg | 2 | 0,045 | 0,035 | 3,7 µg | 1,3 | 0,137 | 0,138 | 11,0 µg | 1,0 | |
| | 9 mg | 3 | 0,036 | 0,027 | 2,9 µg | 1,3 | 0,153 | 0,164 | 12,2 µg | 0,9 | |
| GMP/3 | 10 mg | 1 | 0,050 | 0,042 | 4,0 µg | 1,2 | 0,245 | 0,246 | 19,6 µg | 0,9 | |
| | 10 mg | 2 | 0,033 | 0,022 | 2,6 µg | 1,5 | 0,397 | 0,398 | 31,8 µg | 1,0 | |
| | 10 mg | 3 | 0,042 | 0,030 | 3,4 µg | 1,4 | 0,278 | 0,282 | 22,2 µg | 0,9 | |
| GMP/4 | 10 mg | 1 | 0,065 | 0,056 | 0,7 µg | 1,2 | 0,135 | 0,142 | 11,0 µg | 1,0 | |
| | 11 mg | 2 | 0,071 | 0,142 | 2,4 µg | 0,5 | 0,140 | 0,142 | 11,2 µg | 1,0 | |
| | 10 mg | 3 | 0,066 | 0,051 | 1,7 µg | 1,3 | 0,130 | 0,130 | 10,4 µg | 1,0 | |

| | | | 2. Eluat 1:8 | | | | 3. 1:4 Eluat | | | | |
|---|---|---|---|---|---|---|---|---|---|---|---|
| | | | 260 nm | 280 nm | Ausbeute | 260/28 0 | 260 nm | 280 nm | Ausbeute | 260/28 0 | Σ Eluate |
| GMP/2 | | 1 | 0,099 | 0,101 | 7,9 µg | 1,0 | 0,057 | 0,062 | 2,3 µg | 0,9 | 23,9 µg |
| | | 2 | 0,078 | 0,076 | 6,2µg | 1,0 | 0,041 | 0,049 | 1,6 µg | 0,8 | 18,8 µg |
| | | 3 | 0,103 | 0,112 | 8,2 µg | 0,9 | fehlt | | | | |
| GMP/3 | | 1 | 0,147 | 0,147 | 11,8 µg | 1,0 | 0,084 | 0,098 | 3,4 µg | 0,9 | 34,8 µg |
| | | 2 | 0,256 | 0,252 | 20,5 µg | 1,0 | 0,042 | 0,043 | 1,7 µg | 1,0 | 54,0 µg |
| | | 3 | 0,147 | 0,143 | 11,8 µg | 1,0 | 0,073 | 0,093 | 2,9 µg | 0,8 | 36,9 µg |
| GMP/4 | | 1 | 0,106 | 0,108 | 8,5 µg | 1,0 | 0,083 | 0,098 | 3,3 µg | 0,8 | 22,8 µg |
| | | 2 | 0,111 | 0,114 | 8,9 µg | 1,0 | 0,054 | 0,063 | 2,2 µg | 0,9 | 22,3 µg |
| | | 3 | 0,135 | 0,141 | 10,8 µg | 1,0 | 0,077 | 0,095 | 3,1 µg | 0,8 | 24,3 µg |

Die 1. Eluate waren noch leicht gelb gefärbt und teilweise mit feinen Magnetpartikeln kontaminiert.

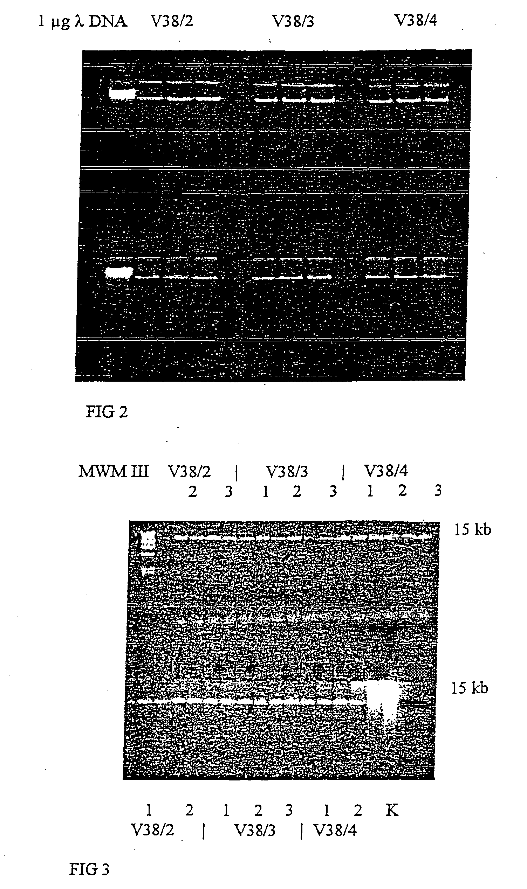

Die Analyse der Eluate im Agarosegel (FIG. 2) zeigt eine gute Reproduzierbarkeit der Ausbeute. Die Magnetpartikel GMP/2 - 4 zeigen keine signifikanten Unterschiede. In den Eluaten 1 (oben) und 2 (unten) ist etwa die gleiche Nukleinsäurekonzentration vorzufinden (vom Gel geschätzt). Eluat 3 zeigt nur noch eine geringe Nukleinsäurekonzentration. In den Überständen ist ebenfalls nur eine geringe Nukleinsäurekonzentration zu beobachten.

Die Expand™ PCR liefert mit allen Proben, bis auf wenige Ausreißer (Tabelle 2), durchweg gute und spezifische Amplifikationsprodukte. Mit den magnetischen Glasbeads ließen sich aus humanen Blutproben Nukleinsäuren isolieren, die in einer anschließenden PCR spezifische Amplifikate lieferten.

**Tabelle 2.**

| Ergebnisse Expand™ PCR *3. Eluat | | | | | |
|---|---|---|---|---|---|
| | 15 kb Expand™ PCR humanes tPA Gen | | | | |
| | | 1. Eluat | | 2. Eluat | |
| GMP/2 | 1 | fehlt | | + | + |
| | 2 | + | + | + | + |
| | 3 | + | + | fehlt | |
| GMP/3 | 1 | + | + | + | + |
| | 2 | (+) | + | + | + |
| | 3 | - | (+) | + | + |
| GMP/4 | 1 | + | + | + | + |
| | 2 | + | + | + | (+)* |
| | 3 | + | + | fehlt | |
| K, BM Kontroll DNA | | | | | |

In FIG. 3 ist ein Gel mit den Reaktionsprodukten nach PCR-Amplifikation gezeigt. MWM III ist ein Molekulargewichtsmarker (Eluat 1, oben; Eluat 2, unten).

### Beispiel 4

### Bindung von DNA-Längenstandard an magnetische Glaspartikel

### 1. Vorbereitung der magnetischen Glaspartikel

Von der Glasmagnetcharge GMP4 wurden 12 mg in Eppendorf-Reaktionsgefäße vorgelegt.

### 2. Lyse und Bindung

In einem 1,5 ml Eppendorf-Gefäß mit 12 mg magnetischen Glaspartikeln werden 900 µl Lysis-Puffer (4.6 M GuSCN, 45 mM Tris, 20 mM EDTA, pH 7,3) und 100 µl DNA-Probe, in der modellhaft DNA-Längenstandard III von Boehringer Mannheim (Katalog-Nr. 528552) eingesetzt wurde, 2 bis 10 sec. gemischt, bis eine homogene Suspension entsteht. Die Lösung wird 20 min bei Raumtemperatur inkubiert, wobei alle 5 min gemischt wird.

Die Magnetseparation erfogt für mindestens 15 sec in einem Magnetpartikelseparator. Der Überstand wird abpipettiert.

### 3. Waschen und Trocknen

Die magnetischen Glaspartikel werden zweimal mit Waschpuffer (5.2 M GuSCN, 50 mM Tris, pH 6.5) zweimal mit 70 % vorgekühltem Ethanol und einmal mit Aceton gewaschen, indem das Magnetfeld entfernt, 800 µl Lösung zupipettiert, 2 sec gemischt, 1 min bei RT inkubiert, das Magnetfeld angelegt und der Überstand schließlich abpipettiert wird.

Nach Entfernung des Acetons werden die Partikel 10 min bei 56 °C im Heizblock bei offenem Deckel getrocknet.

### 4. Elution der DNA

Die DNA wird mit 4x 50 µl Elutionspuffer (10mM Tris-HCl, 1 mM EDTA, pH 8.0) eluiert, indem 10 min unter mehrmaligem Schütteln bei 56 °C inkubiert wird und der DNA-haltige Überstand schließlich in ein neues Eppendorf-Gefäß transferiert wird.

### 5. Analyse der Eluate

Ein Fünftel des Eluatvolumens wurde mit Probenpuffer versetzt und die DNA auf einem 1 %igen Agarosegel bei 90 V aufgetrennt. Zur Bestimmung der Recovery wurde eine Verdünnungsreihe von DNA-Längenstandard III auf das gleiche Gel aufgetragen, die die in den Proben zu erwartenden DNA-Mengen enthält.

Die quantitative Auswertung erfolgte durch Scannen eines Polaroidphotos des Agarosegels. Hierbei wurde die Verdünnungsreihe des Standards als Kalibrator verwendet.

Die Ausbeute an DNA mit magnetischen Glaspartikeln ist in Tabelle 1 dargestellt

**Tabelle 1.**

| Ausbeute an DNA-Längenstandard III mit magnetischen Glaspartikeln | | | | | | | | |
|---|---|---|---|---|---|---|---|---|
| Stande Nr. | DNA - Menge im Stan dard [ng] | Helligkeitsintens Standard (Meßwert) [rel.Einheiten] | Probe Nr | Pigment/ Besd-Typ | Helligkeitsintens Probe (Meßwert) [rel.Einheiten] | errechnete DNA-Menge auf Gel [ng] | errechnete DNA-Mengein [%] Probe [ng] | Recovery |
| 1 | 200 | 65 | 1 | GMP4 | 45 | 139 | 695 | 69,5 |
| 2 | 175 | 56 | 2 | GMP4 | 39 | 120 | 600 | 60,0 |
| 3 | 150 | 51 | | | | | | |
| 4 | 125 | 44 | | | | | | |
| 5 | 100 | 37 | | | | | | |
| 6 | 75 | 25 | | | | | | |
| 7 | 50 | 17 | | | | | | |
| 8 | 25 | 9 | | | | | | |
| 9 | 10 | 4 | | | | | | |

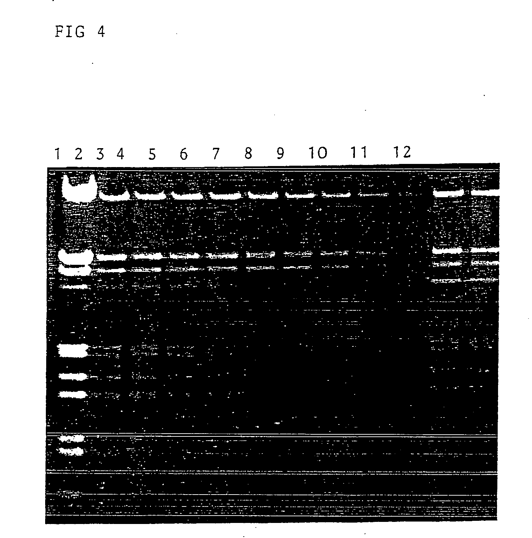

Das Agarosegel, das als Grundlage für die quantitative Auswertung diente, ist in FIG. 4 dargestellt. Es handelt sich um ein 1 %iges Ethidiumbromidgefärbtes Agarosegel. Spur 1 bis 10 entspricht einer Verdünnungsreihe von DNA-Längenstandard III. 1: 1 µg DNA, 2: 200 ng DNA, 3: 175 ng DNA, 4: 150 ng DNA, 5: 125 ng DNA, 6: 100 ng DNA, 7: 75 ng DNA, 8: 50 ng NDA, 9: 25 ng DNA, 10: 10 ng DNA.

Spur 11 und 12 entspricht der von den magnetischen Glaspartikeln eluierten DNA bei Einsatz von 200 ng DNA-Längenstandard.

Im Folgenden sind bevorzugte Ausführungsformen der Erfindung als Bestandteil der Beschreibung dargestellt:
1. Magnetische Partikel mit einer äußeren Glasoberfläche, die im wesentlichen porenfrei ist oder Poren eines Durchmessers von weniger als 10 nm aufweist.
2. Ferromagnetische Partikel mit einer Glasoberfläche.
3. Partikel gemäß Ausführungsform 1 oder 2, dadurch gekennzeichnet, dass sie eine Korngröße von zwischen 10 und 60 µm haben.
4. Partikel gemäß Ausführungsform 1 oder 2, dadurch gekennzeichnet, dass eventuell in der Oberfläche vorhandene Poren einen Durchmesser von weniger als 1 nm haben.
5. Partikel gemäß Ausführungsform 1 oder 2, dadurch gekennzeichnet, dass die Partikel einen Verbundstoff aus einem Kern aus Glimmer und darauf immobilisierten Magnetitpartikeln enthalten, wobei dieser Verbundstoff von einer Glasschicht umschlossen ist.
6. Verfahren zur Isolierung eines biologischen Materials durch
   - Inkontaktbringen einer Probe, die das biologische Material in einer Flüssigkeit enthält, mit Partikeln gemäß einem der Ansprüche 1 bis 5 unter Bedingungen, bei denen das biologische Material an die Partikeloberfläche bindet, und
   - Abtrennung des biologischen Materials von der Flüssigkeit.
7. Verfahren gemäß Ausführungsform 6, dadurch gekennzeichnet, dass es sich bei dem biologischen Material um Nukleinsäuren handelt.
8. Verfahren zur Isolierung von Nukleinsäuren durch
   - Inkontaktbringen einer Probe, welche die Nukleinsäure in nativer Form in einer Flüssigkeit enthält, mit magnetischen Partikeln mit Glasoberflächen unter Bedingungen, bei denen die Nukleinsäuren in nativer Form an die Glasoberfläche binden können und
   - Abtrennung der gebundenen Nukleinsäuren von der Flüssigkeit.
9. Verfahren gemäß einem der Ausführungsformen 6 bis 8, dadurch gekennzeichnet, dass die Abtrennung mit Hilfe eines Magneten geschieht.
10. Verfahren gemäß einem der Ausführungsformen 6 bis 9, dadurch gekennzeichnet, dass die magnetischen Partikel beim Inkontaktbringen mit der Probe nicht vormagnetisiert sind.
11. Verfahren zur Herstellung magnetischer Glaspartikel durch
   - Bereitstellung eines magnetischen Kernes und
   - Umschließen des magnetischen Kernes mit einer im wesentlichen porenfreien Glasoberfläche.
12. Verfahren gemäß Ausführungsform 11, dadurch gekennzeichnet, dass das Umschließen die Abscheidung eines Sols auf der Oberfläche und das anschließende Verdichten des Sols beinhaltet.
13. Verwendung ferromagnetischer Glaspartikel zur Isolierung von Nukleinsäuren in nativer Form.
14. Verwendung magnetischer Glaspartikel zur Isolierung von Nukleinsäuren.

## Patentansprüche

1. Reagenzienzusammensetzung zur Isolierung von Nukleinsäuren aus einer Probe, umfassend
(a) magnetische Partikel mit einer Glasoberfläche,
(b) eine chaotrope Salzlösung,
(c) gegebenenfalls eine Waschlösung und
(d) gegebenenfalls einen Elutionspuffer.

2. Zusammensetzung nach Anspruch 1,
**dadurch gekennzeichnet,**
**dass** die magnetischen Partikel eine durchschnittliche Korngröße von weniger als 100 µm aufweisen.

3. Zusammensetzung nach Anspruch 1 oder 2,
**dadurch gekennzeichnet,**
**dass** die magnetischen Partikel einen inneren Kern z.B. aus einem Verbundstoff oder einem Eisenkern enthalten, auf den die äußere Glasoberfläche aufgebracht ist.

4. Zusammensetzung nach Anspruch 3,
**dadurch gekennzeichnet,**
**dass** der Kern aus einer kristallinen oder keramischen oder glasartigen Struktur besteht, in die Eisenoxid eingelagert ist.

5. Zusammensetzung nach Anspruch 3 oder 4,
**dadurch gekennzeichnet, dass** der Kern aus Magnetit (Fe₃O₄) oder Fe₂O₃ besteht.

6. Zusammensetzung nach einem der Ansprüche 1 bis 5,
**dadurch gekennzeichnet,**
**dass** die magnetischen Partikel ferromagnetisch sind.

7. Zusammensetzung nach einem der Ansprüche 1 bis 6,
**dadurch gekennzeichnet,**
**dass** die Konzentration chaotroper Salze 2-8 mol/l, vorzugsweise 4-6 mol/l beträgt.

8. Zusammensetzung nach einem der Ansprüche 1 bis 7,
**dadurch gekennzeichnet,**
**dass** die chaotropen Salze ausgewählt werden aus Natriumjodid, Natriumperchlorat, Guanidiniumthiocyanat, Guanidiniumisothiocyanat und Guanidiniumhydrochlorid.

9. Zusammensetzung nach einem der Ansprüche 1 bis 8,
**dadurch gekennzeichnet,**
**dass** der Elutionspuffer einen niedrigem Salzgehalt besitzt, vorzugsweise einen Salzgehalt von weniger als 0,2 mol/l.

10. Zusammensetzung nach einem der Ansprüche 1 bis 9,
**dadurch gekennzeichnet,**
**dass** der Elutionspuffer Tris enthält oder entmineralisiertes Wasser ist.

11. Verwendung der Reagenzienzusammensetzung nach einem der Ansprüche 1 bis 10 zur Isolierung der Nukleinsäuren in nativer Form aus einer Probe.

12. Verwendung nach Anspruch 11,
**dadurch gekennzeichnet,**
**dass** es sich bei der Probe um eine klinische Probe handelt.

13. Verwendung nach Anspruch 11 oder 12,
**dadurch gekennzeichnet,**
**dass** die Probe aus Blut, Serum, Mundspülflüssigkeit, Urin, Zerebralflüssigkeit, Sputum, Stuhl, Punktaten und Knochenmarkproben ausgewählt wird.

14. Verwendung nach Anspruch 11,
**dadurch gekennzeichnet,**
**dass** es sich bei der Probe um eine Probe aus dem Bereich der Umweltanalytik, der Lebensmittelanalytik oder der molekularbiologischen Forschung handelt, vorzugsweise um eine Probe aus Bakterienkulturen, Phagenlysaten oder Produkten von Amplifikationsverfahren, z B. PCR.

15. Verwendung nach einem der Ansprüche 11 bis 14,
**dadurch gekennzeichnet;**
**dass** die Nukleinsäuren als Substrat für eine enzymatischen Reaktion eingesetzt werden.

16. Verwendung nach einem der Ansprüche 11 bis 15,
**dadurch gekennzeichnet,**
**dass** die Nukleinsäuren als Substrat zur Sequenzierung, radioaktiven oder nicht- radioaktiven Markierung, Amplifikation einer oder mehrerer Sequenzen, Transkription, Hybridisierung mit markierten Sondennukleinsäuren, Translation oder Ligation eingesetzt werden.
